# EUROPÄISCHE PATENTSCHRIFT

(11) **EP 1 106 143 B1**
(45) Veröffentlichungstag und Bekanntmachung des Hinweises auf die Patenterteilung: **26.05.2004**
(21) Anmeldenummer: 99124152.2
(22) Anmeldetag: 02.12.1999
(51) Int. Cl.: A61B 17/16, A61F 2/36

(54) **Prothesensystem**
Prosthetic system
Système prothétique

(43) Veröffentlichungstag der Anmeldung: 13.06.2001
(73) Patentinhaber: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll & Partner

(56) Entgegenhaltungen:
- EP-A- 0 359 097
- WO-A-98/42279
- FR-A- 2 719 464
- US-A- 3 815 599
- US-A- 4 671 275
- US-A- 5 089 004

## Beschreibung

Man verbindet Endoprothesen mit den Enden von Röhrenknochen im allgemeinen dadurch, daß man einen Schaft der Prothese in der Höhlung des Röhrenknochens verankert. Wenn dies zementlos geschehen soll, muß zur Erreichung hinreichender Verankerungsfestigkeit weitgehende Formübereinstimmung zwischen dem Prothesenschaft und der zu seiner Aufnahme bestimmten Knochenhöhlung bestehen. Dazu verwendet man eine Raspel, deren Gestalt mit derjenigen des Prothesenschafts übereinstimmt. Nach dem Eröffnen der Epiphyse des Knochens und ggf. nach dem Durchbohren der epiphysären Spongiosa bis in den Markkanal der Diaphyse stößt man die Raspel in derjenigen Lage in den Knochen ein, die der Prothesenschaft anschließend einnehmen soll. Dabei wird der spongiöse Knochen durch die Raspelwirkung des Instruments gelöst und größtenteils aus dem Knochen entfernt.

Aus der US 4,671,275 ist eine Raspel bekannt, die einen etwa rechteckigen Querschnitt aufweist und nur an den Ecken gezahnt ist. Dazwischen befinden sich glatte, konkav geformte Flächen, die keine Schneid- oder Reißwirkung ausüben, sondern lediglich zu einer Verdrängung des von ihnen angetroffenen Knochenmaterials führen. *Dadurch wird* die Notwendigkeit, Knochenmaterial um den Prothesenschaft zu packen, vermieden. Der Erfindung liegt die Aufgabe zugrunde, ein Prothesensystem bestehend aus Prothese und Raspel zu schaffen, das einen besonders festen Sitz des Prothesenschafts im Knochen ermöglicht. Sie erreicht dies durch die Merkmale des Anspruchs 1 und vorzugsweise diejenigen der Unteransprüche.

Der Erfindung liegt die Überlegung zugrunde, daß die erhöhte Festigkeit des durch den ungezahnten Profilteil der Raspel erzeugten Bereichs der Knochenhöhlung die Sicherheit des Prothesensitzes um so mehr verbessert, je inniger dieser Bereich mit der Oberfläche des Prothesenschaftes zusammenwirkt. Erfindungsgemäß weist der Schaft daher wenigstens eine Längsrippe auf, die in demjenigen Teil des Prothesenschafts angeordnet ist, die dem ungezahnten Profilteil der Raspel entspricht. Obwohl es vorteilhaft ist, wenn diese Rippe sich über die gesamte Länge des Schaftes erstreckt, ist dies nicht unbedingt erforderlich, sofern ein wesentlicher Teil der Schaftlänge erfaßt wird, der vorzugsweise größer als ein Drittel, weiter vorzugsweise größer als zwei Drittel der Schaftlänge ist.

Die Erfindung hat erkannt, daß ein besonders sicherer Prothesensitz dann erreicht wird, wenn die soeben erwähnte Längsrippe des Schafts im Profil der Raspel wenigstens auf einem wesentlichen Teil von deren Länge fehlt. Der Knochenhohlraum, der zur Aufnahme des Schafts von der Raspel geschaffen wird, weist in diesem Falle noch keine Nut an derjenigen Stelle auf, an der sich die Rippe des Schafts befindet. Vielmehr wird diese Nut erst durch die Schaftrippe selbst geschaffen. Beim Einführen des Schafts schneidet sie sich kielartig in das Knochenmaterial ein. Dadurch wird im Knochenraum ein Bett zur Aufnahme des Prothesenschafts geschaffen, in dem dieser durch seine Querschnittsform die Spongiosa zumindest bereichsweise verpreßt und optimalen Knochenkontakt erreicht, der vorzugsweise mehr als 70%, weiter vorzugsweise mehr als 80% der Schaftoberfläche erfaßt.

Die erfindungsgemäße Wirkung wird noch erhöht, wenn der ungezahnte Profilteil der Raspel gegenüber dem entsprechenden Teil des Schafts Untermaß aufweist, so daß die Oberfläche der Knochenhöhlung nach der Implantation des Prothesenschafts diesen mit Vorspannung umgibt.

Besondere Bedeutung hat die Erfindung für die Verankerung einer zementfrei zu implantierenden Hüftprothese. Wenn deren Schaft angenähert dem Adamschen Bogen gekrümmt ist, sollte der ungezahnte Profilteil auf der ventralen und/oder dorsalen Seite der Raspel - am besten auf beiden Seiten - angeordnet sein, während die Raspel zumindest auf der medialen Seite gezahnt ist. Dadurch läßt sich die Oberfläche der Knochenhöhlung sehr genau der Oberflächenform des Schafts im medialen Bereich anpassen. Zum einen ist dies wichtig, weil die Knochenoberfläche dort ziemlich unregelmäßig ist. Zum anderen wünscht man hier eine verhältnismäßig starke Annäherung der Schaftoberfläche an die harte Knochenrinde. Deshalb ist die verbleibende Spongiosaschicht, soweit die Knochenrinde nicht direkt erreicht wird, bereits verhältnismäßig dicht und stellt wenig Spielraum für eine Verdichtung des spongiösen Knochenmaterials zur Verfügung, so daß die Gefahr besteht, daß in Bereichen zu hoher Pressung die Knochenrinde gesprengt würde, wenn man allein auf die Verdrängung des spongiösen Materials vertrauen würde. Aus diesem Grunde sollte die Zahnung der medialen Raspelseite zumindest im proximalen Abschnitt derselben vorgesehen sein. Das ist ein Abschnitt von etwa 4 bis 7 cm Länge an der medialen Seite des Schafts, gemessen vom oberen Ende des Schaftes. Zweckmäßigerweise ist auch die laterale Seite der Raspel zumindest in deren proximalen Abschnitt gezahnt.

Hingegen ist es zweckmäßig, wenn der distale Abschnitt der Raspel auf der medialen und lateralen Seite ohne scharfe Querkanten ausgebildet ist. Er soll nämlich eine Führungsfunktion beim Einschieben der Raspel in den Knochen erfüllen, indem er - vornehmlich im diaphysären Bereich - an der Innenfläche der Knochenrinde entlanggleitet, wobei eine unnötige Verletzung derselben vermieden werden soll.

Die ungezahnt ausgeführten Bereiche auf der ventralen und/oder dorsalen Seite der Raspel erstrecken sich vorzugsweise über die gesamte Länge der Raspel. Beide Seiten des Prothesenschafts können mit der oben erwähnten Längsrippe ausgerüstet sein, die ebenfalls vorzugsweise im wesentlichen über die gesamte Schaftlänge durchlaufend angeordnet ist. Besonders vorteilhaft ist es, wenn diese Rippe durch ein Paar sie beiderseits einfassender Nuten gebildet ist, wodurch für den innigen Kontakt zwischen dem Prothesenschaft und dem verdichteten Knochenmaterial eine große Kontaktfläche geschaffen wird. Wenn im Raspelprofil diese Rippe fehlt, sind dort nur die Nutquerschnitte vorgebildet, deren Bodenflächen im wesentlichen direkt miteinander verbunden sind. Dadurch entstehen auf der ventralen und dorsalen Seite der Raspel ausgedehnte Flächen, die zweckmäßigerweise einen vom distalen Ende her keilförmig zunehmenden Abstand voneinander aufweisen, um beim Einschieben der Raspel in den Knochen das Knochenmaterial, an dem sie entlanggleiten, allmählich zu verdichten.

Den Erfindungsgedanken kann man dahingehend zusammenfassen, daß ein Prothesensystem bestehend aus Prothese und Raspel geschaffen wird, das zur Schaffung der die Prothese aufnehmenden Höhlung nicht vordergründig das Ausräumen von Knochenmaterial verlangt, sondern das Knochenmaterial in situ beläßt, wobei aber durch markante Formunterschiede zwischen Raspeldorn und Prothesenschaft das spongiöse Knochenmaterial beim Einbringen des Prothesenschafts formbildend derart verpreßt wird, daß sich ein übungsstabil fester Sitz der Prothese mit hohem vitalen Formschluß zum Knochen ergibt.

Die Erfindung wird im folgenden näher unter Bezugnahme auf die Zeichnung erläutert, die ein vorteilhaftes Ausführungsbeispiel veranschaulicht. Es zeigen:
- Fig. 1: einen Schnitt in der lateral/medial-Ebene des proximalen Teils eines Oberschenkelknochens;
- Fig. 2: die Schnittansicht gemäß Fig. 1 mit eingesetzter Raspel;
- Fig. 2a bis 2c: Querschnitte durch die Anordnung gemäß Fig. 2 in den strichpunktiert angedeuteten Schnittebenen;
- Fig. 3: die Schnittansicht gemäß Fig. 1 mit eingesetzter Prothese und
- Fig. 3a bis 3c: Querschnitte durch die Anordnung gemäß Fig. 3 in den strichpunktiert angegebenen Schnittebenen.

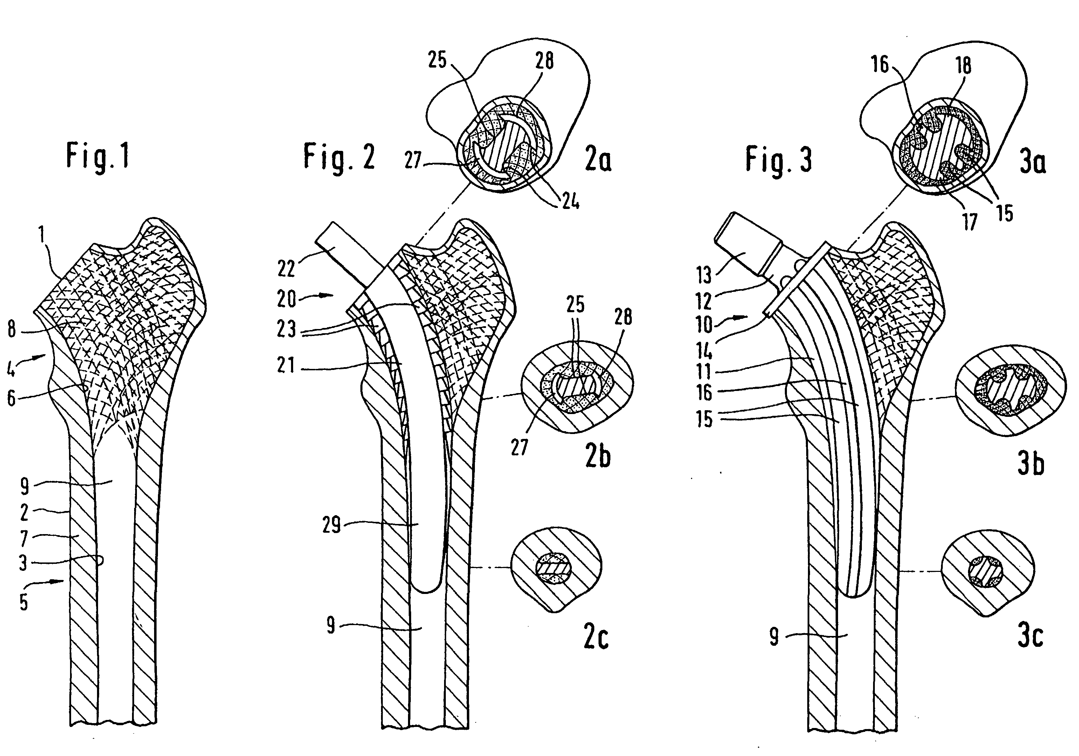

Fig. 1 zeigt das proximale Femurende nach Resektion des Gelenkkopfs längs der Resektionsebene 1. Der Knochen wird nach außen hin von der harten Knochenrinde 7 gebildet, die in der Zeichnung zwischen der Außenfläche 2 des Knochens und der inneren Begrenzungslinie 3 liegt. Im epiphysären Bereich 4 ist die Knochenrinde wesentlich dünner als im diaphysären Bereich 5. Das gilt (abweichend von der schematischen Darstellung der Zeichnung) auch im Bereich des Adamschen Bogens 6, der in der Zeichnung als monotone Bogenlinie gezeichnet ist, aber in Wirklichkeit Unregelmäßigkeiten aufweist. Die Zeichnungslinie 6 stellt demnach eine Annäherung an die Knochenrinde dar.

Im epiphysären Bereich 4 des Knochens ist dessen Innenraum von spongiösem Knochenmaterial 8 gefüllt, das im Markkanal 9 des diaphysären Bereich 5 im wesentlichen fehlt. Beide enthalten weiches Mark. Die Dichte des spongiösen Knochenmaterials nimmt zur Knochenrinde 7 hin zu. Der Begriff Markkanal, wie er in Anspruch 1 verwendet ist, beschränkt sich nicht auf den Hohlraum der Diaphyse, sondern schließt einfachheitshalber auch denjenigen epiphysären Bereich ein, in welchen die Prothese einzusetzen ist.

In sämtlichen Zeichnungen wendet der Knochen dem Betrachter seine ventrale (nach vorne gewendete) Seite zu, sofern es sich um den linken Oberschenkelknochen bzw. die linke Prothese handelt. Die dorsale Seite ist die entgegengesetzte. Die mediale Seite ist in der Zeichnung links und die laterale Seite rechts angeordnet.

Die Endoprothese 10 besteht aus einem Schaft 11, einem Hals 12 und einem Kupplungsstück 13 zur Verbindung mit einem Gelenkkopf. Dieser kann auch einstückig mit dem Hals verbunden sein. Es kann eine Halsauflage 14 vorgesehen sein, die sich auf die Resektionsfläche 1 auflegt.

Der Schaft 11 ist bogenförmig gekrümmt zwischen einer distalen Richtung, die etwa derjenigen der Diaphyse 5 gleicht und einer proximalen Richtung, die der Richtung des natürlichen Kopfhalses nahekommt. Die Krümmung ist im wesentlichen gleichmäßig, so daß der Schaft in einen entsprechend gekrümmten, formgleichen Kanal eingeführt werden kann, der zuvor im epiphysären Bereich des Knochens gebildet wurde. Geringe Abweichungen von der Kreisbogenform können toleriert werden, insbesondere im Hinblick darauf, daß die Dicke des Schafts sich nach distal verringert.

Das Schaftprofil hat einen ovalen Umriß, der in den Querschnittsdarstellungen 3a bis 3c erkennbar ist. Auf der anterioren und posterioren Seite des Schafts sind in das Profil je zwei Nuten 15 eingeschnitten, die zwischen sich eine Rippe 16 begrenzen.

Die Nuten 15 und die Rippe 16 laufen über die gesamte Länge des Schaftes durch, wobei die Dicke der Rippe 16 und die Weite der Nuten etwa konstant bleiben. Der Abstand zwischen dem Nutgrund von einander auf der dorsalen und ventralen Seite gegenüberliegenden Nuten wächst vom distalen bis zum proximalen Ende des Schaftes ein wenig an. Da die Rippe 16 sich im Mittelbereich des Schaftquerschnitts befindet, ragt sie vom Profilkern beträchtlich weiter vor als der mediale 17 und laterale Querschnittsbereich 18 des Schafts. Infolge der Verjüngung des Schafts von dessen proximalen zum distalen Ende hin schwinden diese medialen und lateralen Querschnittsbereiche 17, 18 allmählich nach distal. Die Rippe 16 ist daher sowohl nach medial als auch nach lateral hin großflächig ausgebildet. In der proximalen Hälfte des Prothesenschafts ist die Höhe jeder Rippe 16 im Querschnitt größer als der Abstand der Bodenflächen der sie einfassenden Nuten 15 von der Symmetrieachse. vorzugsweise gilt dies auch im distalen Bereich des Prothesenschafts, wie Fig. 3b und 3c deutlich zeigen.

Die Kontur des medialen Querschnittsbereichs 17 folgt in der proximalen Hälfte der Schaftlänge im wesentlichen einem Kreisbogen (Fig. 3), dessen Krümmungsradius demjenigen des Adamschen Bogens angenähert ist. Es versteht sich, daß mehrere Größenklassen von Prothesen zur Verfügung stehen, die optimale Formanpassung in jeweils einer Größengruppe von Knochen ermöglichen. Die mediale, proximale Schaftoberfläche soll sich am Adamschen Bogen der Knochenrinde bzw. an dem ihr innerlich vorgelagerten, verhältnismäßig dichten spongiösen Knochenmaterial abstützen, das die zur Aufnahme des Prothesenschafts gebildete Knochenhöhle begrenzt.

Zu deren Herstellung ist die in Fig. 2 erkennbare Raspel 20 vorgesehen, die aus einem Schaft 21 und einem Hals 22 besteht. Mit dem Hals und ggf. weiteren, in der Zeichnung nicht erscheinenden Elementen kann sie mit einem Instrumentengriff verbunden werden, der ihre Handhabung gestattet.

Der Schaft 21 der Raspel 20 hat eine Gestalt, die derjenigen des Prothesenschafts 11 ähnlich ist. Beim Vergleich der Fig. 2a und 3a, der Fig. 2b und 3b sowie 2c und 3c erkennt man, daß die medialen und lateralen Abschnitte 17, 18 des Prothesenprofils getreu den Profilabschnitten 27, 28 der Raspel 20, nachgebildet sind. Dies geschieht allerdings zweckmäßigerweise mit einem gewissen Untermaß. Die lateral-mediale Querabmessung der Raspel ist um 0,2 bis 2 mm, vorzugsweise 0,5 bis 1 mm kleiner als die entsprechende Abmessung des Prothesenschafts.

Die Abschnitte 27, 28 des Raspelprofils sind mit einer Zahnung 23 versehen, die zumindest im Bereich des Adamschen Bogens für eine hinreichend definierte Anlagefläche des Prothesenschafts sorgt. Die Zahnung 23 ist auf den proximalen Teil der Raspellänge beschränkt, nämlich auf (gemessen auf der medialen Seite) vorzugsweise mindestens 4, vorzugsweise etwa 5 bis 6 cm. Der verbleibende distale Abschnitt der Raspel, deren Gesamtlänge (gemessen geradlinig zwischen den Mittelpunkten der proximalen und distalen Enden des Raspelschafts) vorzugsweise zwischen 10 und 12 cm liegt, ist ungezahnt, um die Wand des diaphysären Markkanals 9 nicht mehr als notwendig zu verletzen. Außerdem soll dadurch gewährleistet werden, daß die Spitze der Raspel an der Wand des diaphysären Markkanals geführt wird, um die richtige Positionierung der von der Raspel hergestellten Knochenhöhlung im epiphysären Bereich sicherzustellen.

Die an die Profilabschnitte 17, 18 angrenzenden Flanken der Nuten 15 des Prothesenschafts sind bei 24 auch in der Raspel nachgebildet. Es fehlt jedoch die Rippe 16. Mit anderen Worten sind die Bodenflächen der Nuten 15 im Fall der Raspel geradlinig miteinander verbunden zur Bildung einer durchgehenden Bodenfläche 25. Der Abstand zwischen den auf der ventralen und dorsalen Seite der Raspel einander gegenüberliegenden Bodenflächen 25 gleicht etwa dem entsprechenden Abstand der Bodenflächen der Nuten 15, ist aber etwas geringer, nämlich um 0,2 bis 2 mm, vorzugsweise 0,5 bis 1 mm. Die Flächen 25 und Flanken 24 sind ungezahnt, wobei allerdings die Zähne der Profilabschnitte 27, 28 in den Flanken 24 auslaufen.

Der gegenseitige Abstand der Bodenflächen 25 liegt am distalen Ende zwischen 2 und 8 mm und nimmt zum proximalen Ende hin um 3 bis 7 mm zu. Die Bodenflächen 25 sind also keilförmig gestaltet.

Zur Vorbereitung der den Prothesenschaft aufnehmenden Knochenhöhlung wird zunächst ausgehend von derjenigen Stelle der Resektionsfläche 1 des Knochens, an welcher der Mittelpunkt des Halses 12 liegen soll, eine zum diaphysären Markkanal 9 durchgehende Öffnung mittels einer Sonde geschaffen, die das Material nicht ausräumt sondern verdrängt. Dann wird die Raspel 20 in diese Öffnung unter Aufweitung der epiphysären Spongiase eingepreßt. Im letzten Abschnitt dieser Bewegung formen die Zähne 23 hinreichend genaue Anlageflächen für die medialen und lateralen Oberflächen des Prothesenschafts 10. Gleichzeitig wird das spongiöse Knochenmaterial, das von den ungezahnten Flächen 24, 25 der Raspel beaufschlagt wird, verdrängt und verdichtet. Wird anschließend der Prothesenschaft in die Höhlung eingeschoben, so findet er medial und lateral ihn genau positionierende Flächen vor, die infolge des Untermaßes der Raspel eine feste Anlage ermöglichen. Auf den ventralen und dorsalen Seiten schneidet die Rippe 16 kielartig in das dort vorhandene und durch die Raspel verdichtete spongiöse Material ein. Dies wird dadurch weiter verdichtet. Außerdem erfolgt eine weitere Verdichtung dadurch, daß der Nutgrund 25 der Raspel Untermaß gegenüber den entsprechenden Nuten 15 aufweist. Die großflächige Rippe 16 findet dadurch in dem verdichteten Knochenmaterial starken Halt.

Der Prothesenschaft erhält dadurch eine hervorragende Anfangsfestigkeit im Knochen. Damit wird ein postoperativ übungsstabiler Sitz der Prothese in Lastrichtung nach medial wie auch gegen Rotationskräfte gewährleistet. Außerdem wird ein vitaler Verbund der Prothesenoberfläche zum Knochen begünstigt.

Besonders vorteilhaft ist in diesem Zusammenhang, daß die Verpressung des spongiösen Knochenmaterials ermöglicht wird, ohne daß gleichzeitig die Gefahr eines Sprengens der Knochenrinde besteht. Dies beruht zum einen darauf, daß in denjenigen Querschnittsbereichen, in denen eine starke Verdichtung auch zu einer hohen radialen Beanspruchung der Knochenrinde führen würde - nämlich in medialer Richtung - die Raspel gezahnt ist, so daß in diesen Bereichen von der Raspel keine Kompressionswirkung ausgeht. Die Kompression, die von dem Untermaß der Raspel gegenüber dem Prothesenschaft ausgeht, kann genau begrenzt werden. Zum anderen wird die Knochenrinde im ventralen und dorsalen Bereich von den Kompressionskräften dadurch entlastet, daß die keilförmigen, ungezahnten Flächen 25 der Raspel durch die Nutflanken 24 eingefaßt sind und daher von diesen ein Teil der Kompressionskräfte aufgefangen wird. Dies gilt auch beim Einführen des Prothesenschafts in die zuvor von der Raspel geschaffenen Knochenhöhlung. Die Kompressionsfläche, die die Rippe 16 nach außen der Knochenrinde zuwendet, ist verhältnismäßig schmal (nämlich vorzugsweise schmaler als 4 mm), und ihre Kompressionswirkung findet hauptsächlich im Bereich der Nuten 15 statt, ohne daß die Knochenrinde dadurch wesentlich belastet wird.

## Patentansprüche

1. Prothesensystem bestehend aus einer Endoprothese (10) mit einem in den Markkanal einen Knochens einzusetzenden Schaft (11) und einer dem Schaft (11) formähnlichen Raspel (20) zum Vorbereiten einer den Schaft (11) aufnehmenden Höhlung, wobei die Raspel (20) auf einem wesentlichen Teil ihrer Länge einen ungezahnten Profilteil (25) aufweist, **dadurch gekennzeichnet, daß** der Schaft (11) wenigstens eine Längsrippe (16) in demjenigen Teil aufweist, der dem ungezahnten Profilteil (25) der Raspel (20) entspricht und eine der Längsrippe (16) entsprechende Rippe im ungezahnten Profilteil (25) der Raspel (20) wenigstens auf einem wesentlichen Teil der Raspellänge fehlt.

2. Prothesensystem nach Anspruch 1, **dadurch gekennzeichnet, daß** der ungezahnte Profilteil (25) der Raspel (20) gegenüber dem entsprechenden Teil des Schafts (11) Untermaß aufweist.

3. Prothesensystem nach Anspruch 1 oder 2, **dadurch gekennzeichnet, daß** die Endoprothese (10) eine Hüftprothese ist, deren in den Oberschenkelknochen einzusetzender Schaft (11) dem Adamschen Bogen (6) angenähert gekrümmt ist, wobei der ungezahnte Profilteil (25) auf der ventralen und/oder dorsalen Seite der Raspel (20) angeordnet ist.

4. Prothesensystem nach Anspruch 3, **dadurch gekennzeichnet, daß** die Raspel (20) auf der medialen Seite gezahnt (27) ist.

5. Prothesensystem nach Anspruch 3 oder 4, **dadurch gekennzeichnet, daß** die Zahnung (26) auf einen proximalen Abschnitt der Raspel (20) beschränkt ist.

6. Prothesensystem nach einem der Ansprüche 3 bis 5, **dadurch gekennzeichnet, daß** der distale Abschnitt (29) der Raspel (20) auf der medialen und lateralen Seite ohne scharfe Querkanten ausgebildet ist.

7. Prothesensystem nach einem der Ansprüche 3 bis 6, **dadurch gekennzeichnet, daß** die dorsalen und ventralen Seiten (25) der Raspel auf ihrer gesamten Länge ungezahnt sind.

8. Prothesensystem nach Anspruch 1 und 7, **dadurch gekennzeichnet, daß** der Schaft auf seiner ventralen und/oder dorsalen Seite je wenigstens eine Längsrippe (16) aufweist, die im Profil der Raspel fehlt.

9. Prothesensystem nach Anspruch 8, **dadurch gekennzeichnet, daß** die Längsrippe über im wesentlichen die gesamte Schaftlänge durchlaufend angeordnet ist.

10. Prothesensystem nach Anspruch 8 oder 9, **dadurch gekennzeichnet, daß** die Rippe (16) durch sie einfassende Nuten (15) gebildet ist und im Profil der Raspel (20) die Bodenflächen (25) beider Nuten im wesentlichen direkt miteinander verbunden sind.

11. Prothesensystem nach einem der Ansprüche 1 bis 10, **dadurch gekennzeichnet, daß** zwei einander gegenüberliegende, ungezahnte Flächen (25) der Raspel (20) einen vom distalen Ende her keilförmig zunehmenden Abstand voneinander aufweist.

## Claims

1. A prosthesis system consisting of an endoprosthesis (10) with a shank (11) which is to be introduced into the medullary canal of a bone, and of a rasp (20) which is similar in shape to the shank (11) and is used to prepare a cavity for receiving the shank (11), said rasp (20) having, along much of its length, an untoothed profile part (25), **characterized in that** the shank (11) has at least one longitudinal rib (16) **in that** part which corresponds to the untoothed profile part (25) of the rasp (20), and, in the untoothed profile part (25) of the rasp (20),there is no rib corresponding to the longitudinal rib (16), at least over much of the rasp length.

2. The prosthesis system as claimed in claim 1, **characterized in that** the untoothed profile part (25) of the rasp (20) is dimensioned smaller than the corresponding part of the shank (11).

3. The prosthesis system as claimed in claim 1 or 2, **characterized in that**, the endoprosthesis (10) is a hip prosthesis whose shank (11) to be introduced into the femoral bone is curved in approximation to Shenton's line (6), the untoothed profile part (25) being arranged on the ventral and/or dorsal aspect of the rasp (20).

4. The prosthesis system as claimed in claim 3, **characterized in that** the rasp (20) is toothed (27) on the medial aspect.

5. The prosthesis system as claimed in claim 3 or 4, **characterized in that** the toothing (26) is limited to a proximal section of the rasp (20) .

6. The prosthesis system as claimed in one of claims 3 through 5, **characterized in that** the distal section (29) of the rasp (20) is designed without sharp transverse edges on the medial and lateral aspects.

7. The prosthesis system as claimed in one of claims 3 through 6, **characterized in that** the dorsal and ventral aspects (25) of the rasp are untoothed along their entire length.

8. The prosthesis system as claimed in claims 1 and 7, **characterized in that** the shank, on its ventral and/or dorsal aspect, has in each case at least one longitudinal rib (16), which is absent in the profile of the rasp.

9. The prosthesis system as claimed in claim 8, **characterized in that** the longitudinal rib is arranged running along essentially the entire length of the shank.

10. The prosthesis system as claimed in claim 8 or 9, **characterized in that** the rib (16) is formed by grooves (15) enclosing it, and, in the profile of the rasp (20), the bottom surfaces (25) of both grooves are connected essentially directly to each other.

11. The prosthesis system as claimed in one of claims 1 through 10, **characterized in that** two opposite untoothed surfaces (25) of the rasp (20) are spaced apart from each other by a distance which increases in a wedge shape from the direction of the distal end.

## Revendications

1. Système de prothèse constitué d'une endoprothèse (10) avec une tige (11) à insérer dans le canal médullaire d'un os et d'une râpe (20) de forme similaire à la tige (11) pour préparer une cavité recevant la tige (11), la râpe (20) présentant sur une partie essentielle de sa longueur, une partie profilée (25) non dentée, **caractérisé en ce que** la tige (11) présente au moins une nervure longitudinale (16) dans la partie qui correspond à la partie profilée (25) non dentée de la râpe (20), et une nervure correspondant à la nervure longitudinale (16) dans la partie profilée (25) non dentée de la râpe (20) manque au moins sur une partie essentielle de la longueur de la râpe.

2. Système de prothèse selon la revendication 1, **caractérisé en ce que** la partie profilée (25) non dentée de la râpe (20) présente une dimension inférieure à la partie correspondante de la tige (11).

3. Système de prothèse selon la revendication 1 ou 2, **caractérisé en ce que** l'endoprothèse (10) est une prothèse de la hanche dont la tige (11), à insérer dans l'os du fémur, est courbée approximativement comme l'arc d'Adam (6), la partie profilée (25) non dentée étant disposée sur la face ventrale et/ou dorsale de la râpe (20).

4. Système de prothèse selon la revendication 3, **caractérisé en ce que** la râpe (20) est dentée (27) sur le côté médial.

5. Système de prothèse selon la revendication 3 ou 4, **caractérisé en ce que** la denture (26) est limitée à un tronçon proximal de la râpe (20).

6. Système de prothèse selon l'une des revendications 3 à 5, **caractérisé en ce que** le tronçon distal (29) de la râpe (20) est réalisé sur le côté médial et latéral sans arêtes transversales vives.

7. Système de prothèse selon l'une des revendications 3 à 6, **caractérisé en ce que** les faces dorsales et ventrales (25) de la râpe sont non dentées sur toute leur longueur.

8. Système de prothèse selon les revendications 1 et 7, **caractérisé en ce que** la tige présente sur sa face ventrale et/ou dorsale au moins une nervure longitudinale (16) qui manque dans le profil de la râpe.

9. Système de prothèse selon la revendication 8, **caractérisé en ce que** la nervure longitudinale est disposée en continu sur sensiblement toute la longueur de la tige.

10. Système de prothèse selon la revendication 8 ou 9, **caractérisé en ce que** la nervure (16) est formée par des rainures (15) l'entourant et dans le profil de la râpe (20) les surfaces de fond (25) des deux rainures sont reliées entre elles sensiblement directement.

11. Système de prothèse selon l'une des revendications 1 à 10, **caractérisé en ce que** deux surfaces (25) non dentées, opposées l'une à l'autre, de la râpe (20), sont écartées l'une de l'autre d'une distance qui augmente en forme de coin depuis l'extrémité distale.
